# EUROPEAN PATENT APPLICATION

(11) **EP 3 736 761 A1**
(43) Date of publication of application: **11.11.2020**
(21) Application number: 18915195.4
(22) Date of filing: 19.04.2018
(51) Int. Cl.: G06Q 40/08

(54) **INFORMATION PROCESSING DEVICE AND INFORMATION PROCESSING METHOD**

(71) Applicant: Sony Corporation, 108-0075 Tokyo (JP)
(72) Inventor: HIROBE, Keisuke, Tokyo 140-0002 (JP)
(74) Representative: D Young & Co LLP
(86) International application number: PCT/JP2018/016167
(87) International publication number: WO 2019/202707

(57) **Abstract**

To provide an insurance scheme that takes a physical strength and cognitive function into account.

An information processing apparatus including a setting unit configured to set a correlation between an insurance risk and information indicating a physical strength and cognitive function.

## Description

### TECHNICAL FIELD

The present disclosure relates to an information processing apparatus and an information processing method.

### BACKGROUND ART

In recent years, various insurance products such as car insurance and nursing insurance have been provided. When the insurance product is provided, an insurance risk of the user is calculated, and the insurance premium corresponding to the insurance risk is set. There is a need for a technology for calculating the insurance risk more appropriately and setting an appropriate insurance premium for the benefit of both the provider and the user of the insurance product.

For example, Patent Document 1 described below discloses a technology to calculate an insurance risk reflecting driving characteristics regarding car insurance.

### CITATION LIST

### PATENT DOCUMENT

Patent Document 1: Japanese Patent Application Laid-Open No. 2015-225498

### SUMMARY OF THE INVENTION

### PROBLEMS TO BE SOLVED BY THE INVENTION

It is known that the human physical strength and cognitive function declines with advancing age. The physical strength and cognitive function is a concept including physical functions such as muscle strength, endurance, agility, lower limb cooperativeness, and reaction rate, as well as cognitive functions such as memory, attentiveness, judgment, and understanding. The more the physical strength and cognitive function declines, the higher the risk of injury from falls and other causes and the risk of making an error in driving a car become. However, in the existing insurance scheme, the insurance risk is calculated only on the basis of basic information, such as age and gender, and the driving characteristics mentioned in Patent Document 1.

In light of this, the present disclosure provides an insurance scheme that takes a physical strength and cognitive function into account.

### SOLUTIONS TO PROBLEMS

According to the present disclosure, an information processing apparatus including a setting unit configured to set a correlation between an insurance risk and information indicating the physical strength and cognitive function is provided.

Furthermore, according to the present disclosure, an information processing method including setting, by a processor, a correlation between the insurance risk and the information indicating the physical strength and cognitive function is provided.

### EFFECTS OF THE INVENTION

As described above, according to the present disclosure, an insurance scheme that takes a physical strength and cognitive function into account is provided. It is to be noted that the above effects are not necessarily restrictive, and any of the effects presented in the present description or other effects that can be understood from the present description may be achieved together with or in place of the above effects.

### BRIEF DESCRIPTION OF DRAWINGS

Fig. 1 is a view for outlining an information processing system according to an embodiment of the present disclosure.
Fig. 2 is a view for illustrating an example of a measurement method of physical strength and cognitive function information according to the present embodiment.
Fig. 3 is a block diagram presenting an example of a logical configuration of a server according to the present embodiment.
Fig. 4 is a graph for illustrating an example of a method for calculating an insurance risk based on physical strength and cognitive function information according to the present embodiment.
Fig. 5 is a flowchart presenting an example of a flow of setting processing of a correlation between the insurance risk and the physical strength and cognitive function information executed in the server according to the present embodiment.
Fig. 6 is a sequence diagram presenting an example of the flow of insurance risk calculation processing executed in the information processing system according to the present embodiment.
Fig. 7 is a sequence diagram presenting an example of the flow of processing of returning insurance premium executed in the server according to the present embodiment.
Fig. 8 is a block diagram presenting an example of a hardware configuration of the information processing apparatus according to the present embodiment.

### MODE FOR CARRYING OUT THE INVENTION

A preferred embodiment of the present disclosure will be described below in detail with reference to the accompanying drawings. It is to be noted that in the present description and the drawings, components having substantially the same functional configuration are denoted by the same reference numerals, and thus redundant description thereof will be omitted.

It is to be noted that the explanation is given in the following order.
1. Outline of Proposed Technology
   1.1. Outline of Information Processing System
   1.2. Application to Car Insurance
2. Configuration Example
3. Technical Features
   3.1. Calculation of Insurance Risk Based on Physical Strength and Cognitive Function Information
   3.2. Training Support Service
   3.3. Processing Flow
   3.4. Supplement
4. Hardware Configuration Example
5. Summary

### <<1. Outline of Proposed Technology>>

### <1.1. Outline of Information Processing System>

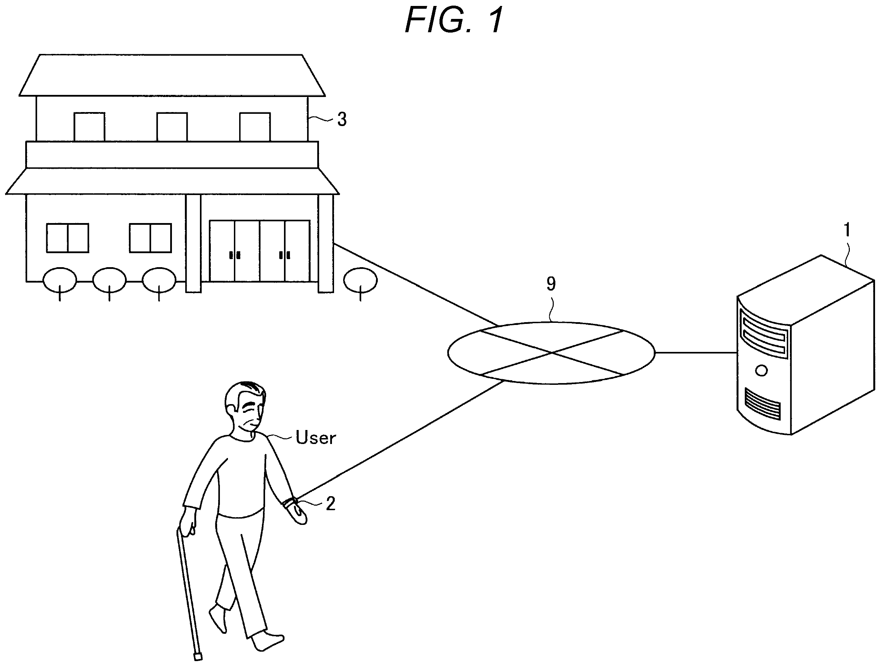

Fig. 1 is a view for outlining the information processing system according to the embodiment of the present disclosure. As presented in Fig. 1, the information processing system according to the present embodiment includes a server 1, a user terminal 2, and a measurement facility 3 (in other words, a measurement apparatus described later). The server 1, the user terminal 2, and the measurement facility 3 are connected via a network 9. A measurement apparatus for measuring physical strength and cognitive function information is provided in the measurement facility 3.

### (1) Measurement Apparatus

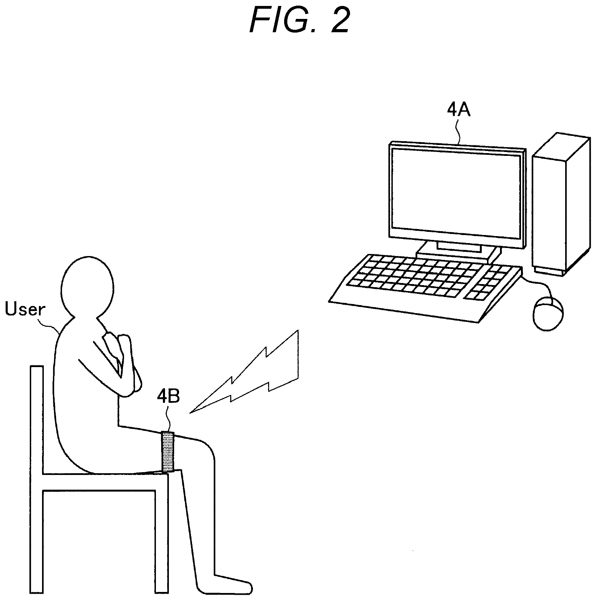

The measurement apparatus provided in the measurement facility 3 is an information processing apparatus that measures the physical strength and cognitive function information of a user (policyholder). The physical strength and cognitive function information is information indicating a physical strength and cognitive function and includes information indicating physical functions and information indicating cognitive functions. The physical functions include muscle strength, endurance, agility, lower limb cooperativeness, reaction rate, and the like. The cognitive functions are memory, attentiveness, judgment, and understanding. The physical strength and cognitive function information is a set of continuous values or discrete values indicating the level of each function such as muscle strength. Measurement of the physical strength and cognitive function information will be described with reference to Fig. 2.

Fig. 2 is a view for illustrating an example of the measurement method of the physical strength and cognitive function information according to the present embodiment. A measurement apparatus 4 according to the present embodiment includes a sensor information processing terminal 4A and a sensor device 4B. The sensor information processing terminal 4A processes the sensor information detected by the sensor device 4B and measures the physical strength and cognitive function information. The sensor information processing terminal 4A is implemented as any information processing apparatus such as a PC, a laptop PC, a tablet terminal, or a smartphone, for example. The sensor device 4B detects information indicating the behavior or state of the user. The sensor device 4B includes, for example, an acceleration sensor, a gyro sensor, a biological information sensor, and the like. The sensor device 4B is implemented as, for example, a wearable device. In the example presented in Fig. 2, the sensor device 4B is attached to the leg of the user. The sensor information processing terminal 4A and the sensor device 4B are connected via a given communication path such as Bluetooth (registered trademark) or Wi-Fi (registered trademark), for example. The sensor information processing terminal 4A and the sensor device 4B may be separated as in the example presented in Fig. 2, or may be provided as a single apparatus.

The sensor information processing terminal 4A displays or audibly outputs a predetermined behavior instruction for measuring the physical strength and cognitive function information. In response to the behavior instruction, the user, for example, repeatedly moves up and down his/her legs while sitting in a chair, repeatedly gets up from and sits down in a chair, or the like. These behaviors are detected by the sensor device 4B attached to the user's leg or the like as presented in Fig. 2, and sensor information indicating the detection result is transmitted to the sensor information processing terminal 4A. The sensor information processing terminal 4A analyzes the sensor information received from the sensor device 4B, and measures (for example, quantifies) and records the physical strength and cognitive function information. Alternatively, the information processing terminal 4A may display a screen for measuring the physical strength and cognitive function information, and measure the physical strength and cognitive function information on the basis of input to the screen by the user. In addition, the sensor information processing terminal 4A transmits the measured cognitive function information to the server 1.

An example of the measurement method for each item of the physical strength and cognitive function information will be described. The "muscle strength" can be quantified by measuring duration time of movement of getting up from and sitting down in a chair and of raising the foot while in a sitting position. The "agility" can be quantified by measuring the number of steps taken to tread in a sitting position. The "cooperativeness" can be quantified by measuring the accuracy of moving the foot in a triangle to the tempo in a sitting position. The "reaction rate" can be quantified by measuring the speed at which the user moves his foot in response to the instruction displayed on the screen in a sitting position. The "endurance" can be quantified by measuring the time of duration of vocalization in a sitting position and the speed drop in the time of movement of getting up from and sitting down in a chair. In addition, the "memory" can be quantified by how well one can memorize items displayed on the screen. The "attentiveness" can be quantified by how quickly and accurately one can press a button in accordance with an instruction on the screen.

### (2) User Terminal

The user terminal 2 is an information processing apparatus attached to or carried by the user. The user terminal can be implemented as a tablet terminal, a smartphone, or a wearable device. The user terminal 2 includes, for example, a biological sensor, a position measurement unit (indoor/outdoor positioning), various sensors such as an acceleration sensor and a gyro sensor, a communication unit enabling communication using any communication standard, an input unit such as a touch screen, and an output unit such as a display.

The user terminal 2 measures (or acquires) information indicating the situation of daily life such as meal content, calorie intake, calorie consumption, number of steps, travel distance, intensity and time of exercise, heart rate, and sleeping hours. That is, the information indicating the situation of daily life includes information indicating the overall life of the user in addition to the so-called activity amount information. The situation of daily life may include the number of steps, exercise, sleeping hours, and activities such as eating. The user terminal 2 may accept input of information indicating the situation of daily life from the user. The user terminal 2 transmits to the server 1 information indicating the situation of daily life having been measured or input.

On the basis of the information received from the server 1, the user terminal 2 displays or audibly outputs a predetermined training instruction for improving the physical strength and cognitive function of the user. The user can improve the physical strength and cognitive function by implementing training on the basis of the training instruction. The state of execution of the training of the user is detected by the user terminal 2 and transmitted to the server 1. It is to be noted that although the user terminal 2 is directly connected to the network 9 in Fig. 1, the user terminal 2 may be connected to the network 9 via the measurement facility 3.

### (3) Server

The server 1 is an information processing apparatus performing various processing related to insurance contracted by the user. For example, the server 1 calculates the insurance risk of the user, calculates the insurance premium reflecting the insurance risk, and calculates the insurance money to be paid to the user. In particular, the server 1 calculates the insurance risk on the basis of the physical strength and cognitive function information of the user.

The insurance risk is an indicator of the risk and occurrence probability of an event for which insurance money is paid. The events for which insurance money is paid include, for example, a car accident under car insurance, death under life insurance, and nursing care need certification under nursing insurance. The insurance risk is reflected in the insurance premium. Typically, the higher the insurance risk is, the higher the insurance premium becomes, and the lower the insurance risk is, the lower the insurance premium becomes. It is to be noted that the insurance premium is money paid by the user to the insurance company. Furthermore, the insurance money is money paid by the insurance company to the user, for example, when an event occurs or when the contract expires.

Furthermore, the server 1 provides the user with advice such as an instruction of training for improvement of the physical strength and cognitive function of the user.

### <1.2. Application to Car Insurance>

The proposed technology is applicable to any type of insurance such as car insurance, life insurance, or nursing insurance. Hereinafter, car insurance will be described as an example.

In the field of car insurance, there has been a lot of discussion on the ideal form of insurance for the elderly. The human tends to decline in terms of the physical strength and cognitive function with advancing age, and the more the physical strength and cognitive function declines, the higher the risk of occurrence of a car accident. For this reason, it is desirable to establish an insurance scheme in accordance with the physical strength and cognitive function.

On the other hand, there is an opinion that elderly should continue to drive their cars for their health. This is because it is thought that driving a car requires the physical strength and cognitive function and the decline in the physical strength and cognitive function can be suppressed by driving a car on a daily basis. In view of the recent declining birthrate and aging population, the increase in social security cost, etc., it is desirable for the elderly to live as healthily as possible. As an idea for that, it is desirable to establish a scheme that enables the elderly to continue driving the car.

Therefore, the proposed technology provides an insurance scheme that takes the physical strength and cognitive function into account. For example, the server 1 calculates an insurance risk and an insurance premium in accordance with the physical strength and cognitive function, after clarifying the relationship between the insurance risk and the physical strength and cognitive function. This makes it possible to appropriately calculate the insurance risk, especially for the elderly.

Furthermore, the server 1 provides the user with advice such as an instruction of training for improvement of the physical strength and cognitive function. This makes it possible to improve the physical strength and cognitive function of the user. As a result, it is possible to reduce the insurance risk by making it difficult to cause a car accident due to a decline in the physical strength and cognitive function. Furthermore, it is possible to promote the health of the elderly in particular by improving the physical strength and cognitive function.

The outline of the proposed technology has been described above. In the following description, it is assumed that the user is an elderly person, the measurement facility 3 is a nursing facility or a home, and the insurance is car insurance.

### <<2. Configuration Example>>

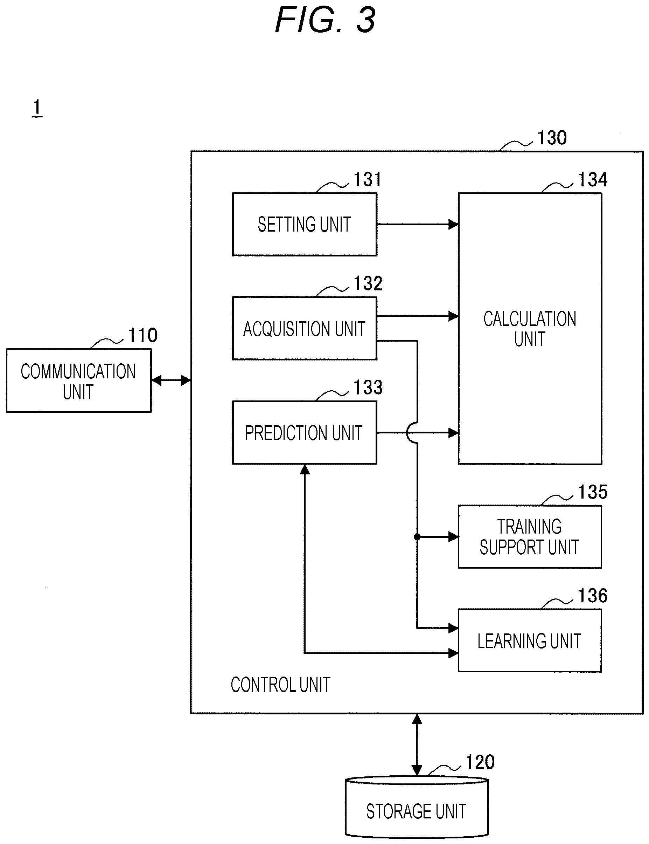

Fig. 3 is a block diagram presenting an example of the logical configuration of the server 1 according to the present embodiment. As presented in Fig. 3, the server 1 includes a communication unit 110, a storage unit 120, and a control unit 130.

### (1) Communication Unit 110

The communication unit 110 transmits and receives information to and from other apparatuses by wired/wireless means. The communication unit 110 is connected to Bluetooth, Wi-Fi, a local area network (LAN), a telephone line, or the like, and communicates with the user terminal 2 and the measurement apparatus 4 via the network 9.

### (2) Storage Unit 120

The storage unit 120 temporarily or permanently stores a program for the operation of the server 1 and various data. The storage unit 120 stores basic information of the user, physical strength and cognitive function information, information indicating the situation of daily life, and various types of information for insurance risk calculation such as a result of implemented training. It is to be noted that the basic information is so-called attribute information such as age, gender, weight, height, body fat percentage, nationality, place of residence, and medical history. The basic information is, for example, input by the user, in advance, and stored in the storage unit 120.

### (3) Control Unit 130

The control unit 130 provides various functions of the server 1. For example, the control unit 130 includes a setting unit 131, an acquisition unit 132, a prediction unit 133, a calculation unit 134, a training support unit 135, and a learning unit 136. The function of each component will be briefly described below.

The setting unit 131 has a function of performing pre-processing for calculating the insurance risk. More specifically, the setting unit 131 sets a relative relationship between the insurance risk and the physical strength and cognitive function information. The setting unit 131 outputs the setting contents to the calculation unit 134.

The acquisition unit 132 has a function of acquiring the physical strength and cognitive function information of the user. For example, the acquisition unit 132 acquires the measurement result of the physical strength and cognitive function information from the measurement apparatus 4. The acquisition unit 132 may transmit, to the measurement apparatus 4, a behavior instruction for measuring the physical strength and cognitive function information. In addition, the acquisition unit 132 may also notify the user of a notification that prompts the user to measure the physical strength and cognitive function information. The acquisition unit 132 outputs the acquired physical strength and cognitive function information to the calculation unit 134, the training support unit 135, and the learning unit 136, and accumulates the information in the storage unit 120.

The prediction unit 133 has a function of predicting the future physical strength and cognitive function information of the user. The prediction unit 133 predicts future physical strength and cognitive function information on the basis of, for example, the user's current physical strength and cognitive function information, history of physical strength and cognitive function information, history of the result of the implemented training, basic information, and/or information indicating the situation of daily life. The prediction unit 133 outputs the prediction result to the calculation unit 134 and the learning unit 136. It is to be noted that a prediction model such as deep learning can be used for prediction by the prediction unit 133.

The calculation unit 134 has a function of performing various calculations related to the insurance of the user. The calculation unit 134 calculates the insurance risk of the user and calculates an insurance premium on the basis of the insurance risk. The calculation result by the calculation unit 134 can be notified to the user.

The training support unit 135 (corresponds to a generation unit) provides a service that supports the implementation of training of the user for improvement of the physical strength and cognitive function on the basis of the physical strength and cognitive function information of the user. For example, the training support unit 135 generates a training menu (training items, intensity, frequency, and so on), notifies the user of the training menu, and records of the user's implementation result.

The learning unit 136 has a function of learning for improving the prediction accuracy of the prediction unit 133. The learning unit 136 learns the parameters of the prediction model used by the prediction unit 133 and outputs the learning result to the prediction unit 133, thereby updating the prediction model. The learning data is physical strength and cognitive function information of a plurality of users, history of the physical strength and cognitive function information, history of the result of the implemented training, basic information, and/or information indicating the situation of daily life. The learning unit 136 may update the prediction model of the prediction unit 133 on the basis of the difference between the physical strength and cognitive function information predicted by the prediction unit 133 and the physical strength and cognitive function information actually acquired subsequently by the acquisition unit 132. The learning unit 136 may learn the prediction model every time the difference is obtained. Such additional learning allows the prediction accuracy to be further improved.

### <<3. Technical Features>>

### <3.1. Calculation of Insurance Risk Based on Physical Strength and Cognitive Function Information>

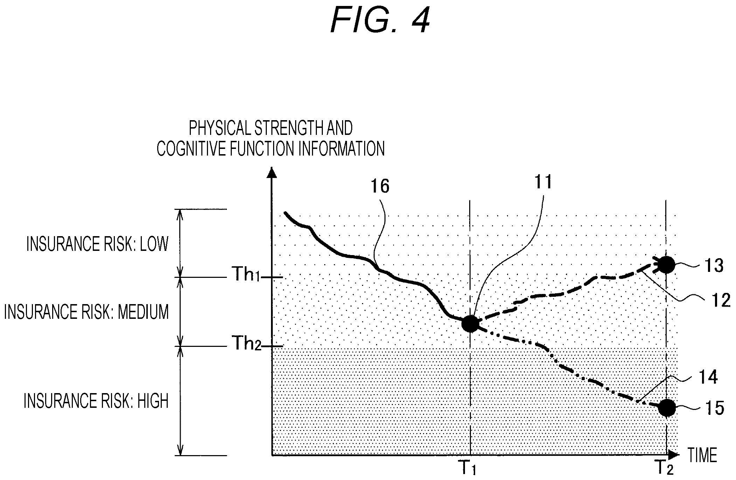

Hereinafter, a method for calculating an insurance risk based on physical strength and cognitive function information will be described with reference to Fig. 4. Fig. 4 is a graph for illustrating an example of the method for calculating an insurance risk based on physical strength and cognitive function information according to the present embodiment. The vertical axis of this graph represents physical strength and cognitive function information. Here, for the sake of simplicity of explanation, let the physical strength and cognitive function information be a single-axis scalar value, and the higher the value is, the higher the physical strength and cognitive function is (that is, good), and the lower the value is, the lower the physical strength and cognitive function is (that is, bad). The horizontal axis of this graph represents time, and time flows from left to right.

### (1) Setting of Relative Relationship between Insurance Risk and Physical Strength and Cognitive Function Information

The server 1 (for example, the setting unit 131) sets the correlation between the insurance risk and the physical strength and cognitive function information. The correlation here is information indicating a correspondence relationship between the degree of physical strength and cognitive function information and the degree of the insurance risk. The correlation may be taken as a model (function) in which the physical strength and cognitive function information is the input and the insurance risk is the output. For example, the server 1 sets a correlation by statistically processing the physical strength and cognitive function information of a plurality of users and the presence/absence of occurrence of car accidents and the results of the accident details of the plurality of users. It is to be noted that a plurality of users who is the acquisition sources of the results used for setting the correlation is typically different from the users for whom the insurance risk and insurance premium are calculated.

The server 1 may set the correlation on the basis of the basic information of the user. For example, the server 1 sets a correlation by statistically processing the physical strength cognitive information of other users having basic information similar to the basic information of the user, and the presence/absence of occurrence of car accidents and the results of the accident details of the other users. The server 1 may establish, in advance, a correlation (for example, correlation for elder male) for each category of the basic information and set the correlation of the category corresponding to the basic information of the user.

Here, setting the correlation in the graph presented in Fig. 4 corresponds to setting threshold values Th₁ and Th₂ of the physical strength and cognitive function information that classifies low, medium, and high insurance risks.

By setting the correlation between the insurance risk and the physical strength and cognitive function information, it becomes possible to calculate the insurance risk and the insurance premium based on the physical strength and cognitive function information of the user as described later.

### (2) Calculation of Insurance Risk

The server 1 (for example, the calculation unit 134) calculates an insurance risk. An example of the method for calculating an insurance risk will be described below. It is to be noted that the server 1 may calculate the insurance risk by combining a plurality of methods for calculation described below.

### - Calculation of insurance risk based on current physical strength and cognitive function information

The server 1 (for example, the calculation unit 134) calculates the insurance risk of the user on the basis of the current physical strength and cognitive function information of the user for whom the insurance risk is calculated and the correlation described above. For example, in the example presented in Fig. 4, the server 1 calculates that the insurance risk is low if the physical strength and cognitive function information is equal to or greater than the threshold value Th₁, calculates that the insurance risk is medium if the physical strength and cognitive function information is equal to or greater than the threshold value Th₂ and less than the threshold value Th₁, and calculates that the insurance risk is high if the physical strength and cognitive function information is less than the threshold value Th₂. As presented in Fig. 4, since physical strength and cognitive function information 11 at the current time T₁ is equal to or greater than the threshold value Th₂ and less than the threshold value Th₁, the server 1 calculates that the insurance risk of the user is medium.

Thus, the insurance risk is calculated on the basis of the current physical strength and cognitive function information of the user, whereby the insurance risk corresponding to the current physical strength and cognitive function of the user can be grasped. In particular, an increase in risk of a car accident due to the decline in the physical strength and cognitive function of the elderly person can be appropriately reflected in the insurance risk.

### - Calculation of insurance risk based on prediction result of future physical strength and cognitive function information

The server 1 (for example, the prediction unit 133) predicts future physical strength and cognitive function information of the user. The server 1 obtains future physical strength and cognitive function information by inputting into the prediction model, for example, the current physical strength and cognitive function information of the user, history of the physical strength and cognitive function information, history of the result of the implemented training, basic information, and/or information indicating the situation of daily life.

The server 1 (for example, the calculation unit 134) calculates the insurance risk of the user on the basis of the prediction result of the future physical strength and cognitive function information of the user for whom the insurance risk is calculated and the correlation described above. As the method for calculating a future insurance risk of the user, a method similar to the method for calculating the insurance risk based on the current physical strength and cognitive function information can be used. This makes it possible to calculate the future insurance risk in anticipation of the degree of decline or the degree of improvement of the future physical strength and cognitive function of the user.

The server 1 calculates the insurance risk of the user on the basis of the prediction result of the physical strength and cognitive function information of the user corresponding to the implementation assumption of the training of the user for improvement of the physical strength and cognitive function. The implementation assumption of the training is an assumption of the load of the imposed training and the degree of implementation of the imposed training (frequency, completion rate, and so on). The higher the implementation assumption is (that is, the load is higher and/or the degree of implementation is higher), the higher the improvement effect of the training on the physical strength and cognitive function is, and as a result, the insurance risk is predicted to decline significantly in the future. On the other hand, the lower the implementation assumption is (that is, the load is lower and/or the degree of implementation is lower), the lower the improvement effect of the training on the physical strength and cognitive function is, and as a result, the insurance risk is predicted not to decline significantly in the future. In this regard, the future insurance risk can be calculated with better accuracy by calculating the insurance risk taking the implementation assumption of training into account. Furthermore, the server 1 may present to the user an implementation assumption of training and the insurance risk calculated on the basis of the implementation assumption in association with each other. This makes it possible to prompt the user to implement the training. Furthermore, the server 1 may present to the user a plurality of implementation assumptions of training and the insurance risks calculated on the basis of the plurality of implementation assumptions in association with each other. Since it is clearly presented to the user that the higher the implementation assumption of training is, the lower the future insurance risk is, it is possible to prompt the user to increase the load of the training and to improve the degree of implementation.

As an example of the plurality of implementation assumptions, the server 1 may calculate the insurance risk of the user on the basis of the prediction result of the physical strength and cognitive function information of the user in each of the cases where the user implements training for improvement of the physical strength and cognitive function and where the user does not do so. In the example presented in Fig. 4, with reference to a prediction result 12 of the physical strength and cognitive function information in a case where the user implements training, the server 1 calculates that the insurance risk at a future time T₂ is low because physical strength and cognitive function information 13 at the future time T₂ is equal to or greater than the threshold value Th₁. In addition, with reference to a prediction result 14 of the physical strength and cognitive function information in a case where the user does not implement training, the server 1 calculates that the insurance risk at the future time T₂ is high because physical strength and cognitive function information 15 at the future time T₂ is less than the threshold value Th₂. The server 1 can present each of the calculated insurance risks to the user. Accordingly, it is possible to prompt the user to implement the training by presenting to the user the difference in insurance risk between the case of implementing the training and the case of not implementing the training.

### - Calculation of insurance risk based on history of physical strength and cognitive function information

The server 1 (for example, the calculation unit 134) may calculate the insurance risk of the user on the basis of the history of the physical strength and cognitive function information of the user. The history of the physical strength and cognitive function information of the user is, for example, a time series change of the physical strength and cognitive function information measured from the past to the present. For example, the server 1 can predict the decline speed of the physical strength and cognitive function in the future on the basis of a time series change 16 of the physical strength and cognitive function information of the user presented in Fig. 4, and can calculate the insurance risk of the user on the basis of such prediction. In other words, the history of the physical strength and cognitive function information of the user can be used for prediction of the future physical strength and cognitive function information of the user.

The improvement/decline speed of the physical strength and cognitive function can vary depending on the user. In this regard, an individual difference in the improvement/decline speed of the physical strength and cognitive function can be reflected in the insurance risk by calculating the insurance risk on the basis of the history of the physical strength and cognitive function information of the user.

Furthermore, the server 1 may calculate the insurance risk of the user on the basis of the history of the result of the training implemented by the user. The history of the result of the implemented training is a history of the load of the imposed training and the degree of implementation of the imposed training (frequency, completion rate, and so on). For example, on the basis of the history of the result of the training implemented by the user and the history of the physical strength and cognitive function information, the server 1 predicts the improvement effect of the physical strength and cognitive function by the training imposed on the user in the future. Then, the server 1 calculates the insurance risk of the user on the basis of the prediction. In other words, the history of the result of the training implemented by the user can be used to predict the future physical strength and cognitive function information of the user.

Even in a case where a plurality of users implements the same training at the same degree of implementation, individual differences can occur in the improvement effect of the physical strength and cognitive function. In this regard, an individual difference in the improvement effect of the physical strength and cognitive function by the training can be reflected in the insurance risk by calculating the insurance risk on the basis of the history of the result of the training implemented by the user.

### - Calculation of insurance risk based on basic information

The server 1 (for example, the calculation unit 134) may calculate the insurance risk on the basis of the basic information of the user in addition to the physical strength and cognitive function information.

The insurance risk can be calculated more appropriately by calculating the insurance risk on the basis of the basic information. For example, it is considered that the older one is, the easier the decline in the physical strength and cognitive function leads to a car accident. Accordingly, the insurance risk can be calculated more appropriately by calculating the insurance risk with more weight given to the physical strength and cognitive function information as one gets older. In addition, it is known that the risk of lifestyle-related diseases and the like is high in a case where the body mass index (BMI) calculated from weight and height is too high, and the risk of immune deficiency and the like is high in a case where BMI is too low. Accordingly, the insurance risk corresponding to life insurance, for example, can be appropriately calculated by calculating the insurance risk on the basis of the weight and the height.

### - Calculation of insurance risk based on information indicating situation of daily life

The server 1 (for example, the calculation unit 134) may calculate the insurance risk on the basis of information indicating the situation of daily life. The information indicating the situation of daily life is detected by the user terminal 2, for example, and stored in the storage unit 120.

The insurance risk can be calculated more appropriately by calculating the insurance risk on the basis of the information indicating the situation of daily life. This is because the improvement/decline speed of the physical strength and cognitive function is affected by daily life.

### (3) Setting of Insurance Premium

The server 1 (for example, the calculation unit 134) sets the insurance premium on the basis of the calculated insurance risk. Typically, the higher the insurance risk is, the higher the server 1 sets the insurance premium, and the lower the insurance risk is, the lower the server 1 sets the insurance premium. It is to be noted that, as described above, the insurance risk is calculated on the basis of at least any one of the current physical strength and cognitive function information, the prediction result of the future physical strength and cognitive function information, the history of the physical strength and cognitive function information, the basic information, or the information indicating the situation of daily life.

In other words, the server 1 may graduate the user on the basis of the calculated insurance risk. Typically, the higher the insurance risk is, the lower the server 1 graduates, and the lower the insurance risk is, the higher the server 1 graduates. The graduation is information reflecting the insurance risk of the user, and the insurance premium is discounted or raised in accordance with to the graduation.

The server 1 may discount the insurance premium, in advance, on the basis of a prior commitment to the training for improvement of the physical strength and cognitive function. For example, in a case where the user has pledged, in advance, to implement training for improvement of the physical strength and cognitive function, the server 1 may calculate the insurance risk on the basis of the degree of improvement in the physical strength and cognitive function predicted in a case where the training is implemented, and charge the user an insurance premium corresponding to the insurance risk. This allows the user to receive a discount of the insurance premium before implementing the training. It is to be noted that the prior commitment may be, for example, an application for a training course provided at any facility such as the measurement facility 3 or a health club, or provided by a Web service or the like. In addition, for example, a training course and insurance (with discounted insurance premium) may be sold as a package.

Furthermore, while once setting a higher insurance premium, the server 1 may return a part of the premium for the degree of improvement in the form of discount of insurance premium in a case where an improvement in the physical strength and cognitive function is predicted. For example, the server 1 first sets the insurance premium on the basis of the insurance risk in a case where the user does not implement the training for improvement of the physical strength and cognitive function, and charges the insurance premium to the user. Thereafter, the server 1 calculates the insurance risk in anticipation of future improvement of the physical strength and cognitive function, while acquiring the result of the training implemented by the user for improvement of the physical strength and cognitive function. Then, the server 1 return the user the difference between the insurance risk in anticipation of future improvement of the physical strength and cognitive function and the charged insurance premium.

### <3.2. Training Support Service>

The server 1 (for example, the training support unit 135) provides a service to support the implementation of training of the user for improvement of the physical strength and cognitive function.

The server 1 generates information to instruct the user to implement training for improvement of the physical strength and cognitive function of the user (hereinafter also referred to as training instruction information) on the basis of the physical strength and cognitive function information of the user. The training instruction information is information indicating a training menu to instruct the user to implement, and includes information indicating the type, intensity, frequency, and the like of the training to be implemented by the user, for example. The server 1 generates a training menu of a load suitable for the current physical strength and cognitive function information of the user. The server 1 may generate a training menu estimated to have a high improvement effect for the physical strength and cognitive function for the user on the basis of the history of the result of the training implemented by the user and the history of the physical strength and cognitive function information of the user. This makes it possible to further enhance the improvement effect of the physical strength and cognitive function by the training.

The server 1 transmits the training instruction information to the user terminal 2. The user terminal 2 displays or audibly outputs the training instruction on the basis of the received training instruction information. The user terminal 2 detects the state of the training implemented by the user on the basis of the training instruction, and reports the detection result to the server 1. Then, the server 1 records the result of the implemented training on the basis of the reported information. The server 1 may control the contents of the training such as increasing or decreasing the load of the training on the basis of the record of the result of the implemented training.

By providing such a training support service, the user can work hard on improvement of the physical strength and cognitive function on a daily basis. This makes it possible not only to promote the health of the user but also to reduce the insurance risk by making it difficult to cause an accident.

The training instruction information can include information other than a training instruction for improving the physical strength and cognitive function. For example, the training instruction information may include instructions regarding meals and sleep. This makes it possible to further enhance the effect of promoting the health of the user and making it difficult to cause an accident.

### <3.3. Processing Flow>

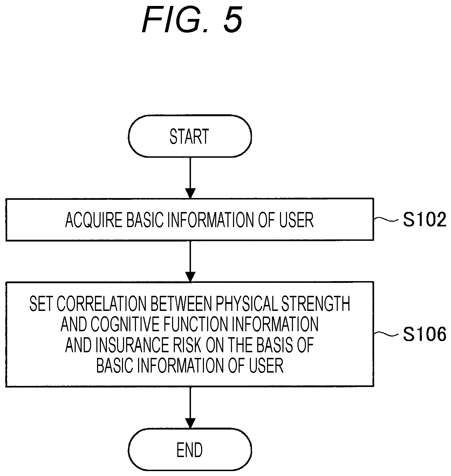

The flow of processing executed in the information processing system according to the present embodiment will be described below with reference to Figs. 5 to 7.

### (1) Pre-processing

Fig. 5 is a flowchart presenting an example of the flow of the setting processing of a correlation between the insurance risk and the physical strength and cognitive function information executed in the server 1 according to the present embodiment. The processing presented in Fig. 5 is executed as pre-processing for calculating the insurance risk.

As presented in Fig. 5, the server 1 first acquires the basic information of the user for whom the insurance risk is calculated (Step S102). Next, the server 1 sets the correlation between the insurance risk and the physical strength and cognitive function information on the basis of the basic information of the user (Step S106) .

### (2) Insurance Risk Calculation Processing

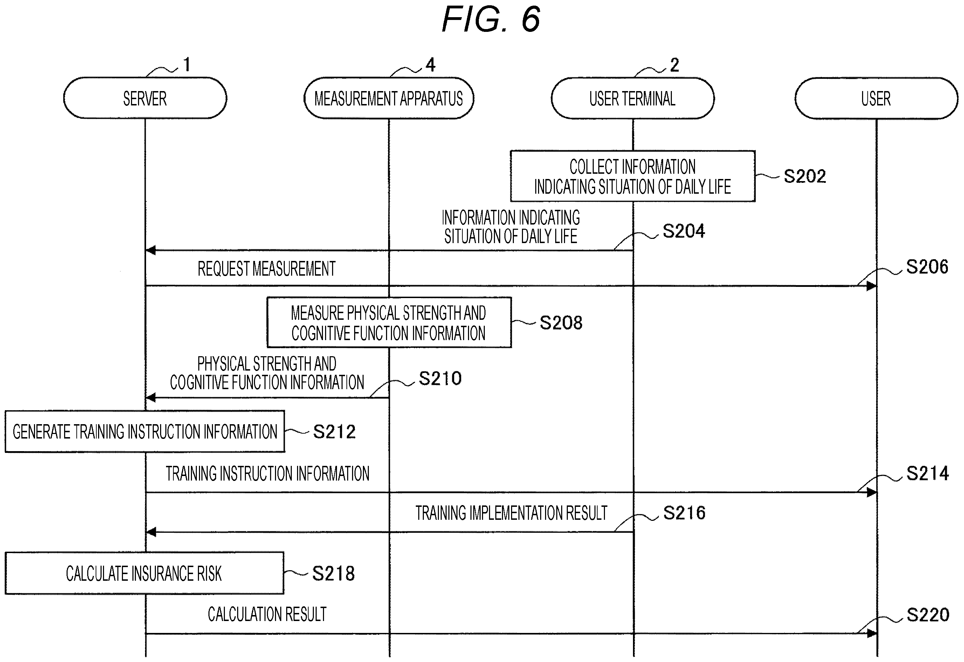

Fig. 6 is a sequence diagram presenting an example of the flow of insurance risk calculation processing executed in the information processing system according to the present embodiment. The server 1, the user terminal 2, and the measurement apparatus 4 are involved in this sequence.

As presented in Fig. 6, the user terminal 2 first collects information indicating the situation of daily life of the user (Step S202), and transmits to the server 1 the collected information indicating the situation of daily life of the user (Step S204). Next, the server 1 notifies the user of a message requesting to measure the physical strength and cognitive function information (Step S206). Such a message may be transmitted to the user terminal 2 and output to the user, or may be notified by any other means such as e-mail. When the user visits the measurement facility 3 on the basis of the notified message, the measurement apparatus 4 measures the physical strength and cognitive function information (Step S208), and transmits the measured physical strength and cognitive function information to the server 1 (Step S210).

The server 1 generates training instruction information for instructing the user to implement training for improving the physical strength and cognitive function of the user on the basis of the received physical strength and cognitive function information (Step S212). Next, the server 1 notifies the user of the generated training instruction information (Step S214). The training instruction information may be transmitted to the user terminal 2 and output to the user, or may be notified by any other means such as e-mail. When the user implements the training on the basis of the training instruction information, the user terminal 2 detects the state of the training of the user and transmits, to the server 1, the detected result of the implemented training (Step S216).

Subsequently, the server 1 calculates the insurance risk (Step S218). For example, the server 1 calculates the insurance risk on the basis of the physical strength and cognitive function information, the result of the implemented training, and the basic information of the user. The server 1 may also calculate the insurance premium and the like on the basis of the calculated insurance risk. Then, the server 1 notifies the user of the calculation result (Step S220). The calculation result may be transmitted to the user terminal 2 and output to the user, or may be notified by any other means such as e-mail.

### (3) Processing of Returning Insurance Premium

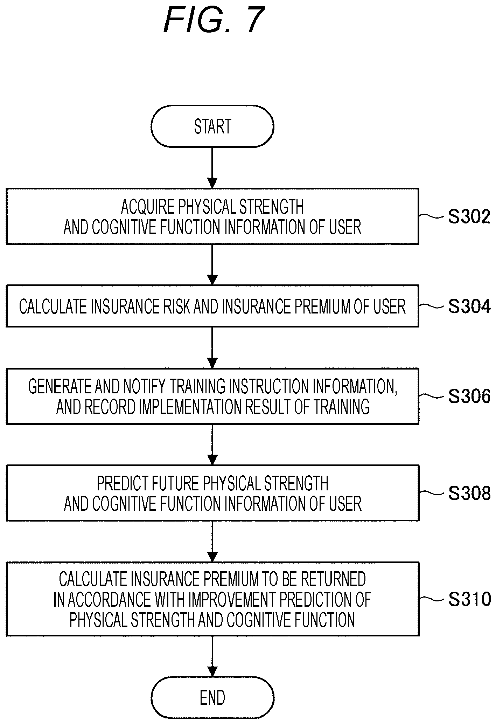

Fig. 7 is a sequence diagram presenting an example of the flow of the processing of returning insurance premium executed in the server 1 according to the present embodiment. As presented in Fig. 7, the server 1 first acquires and records the physical strength and cognitive function information of the user (Step S302). Next, the server 1 calculates the insurance risk and the insurance premium of the user on the basis of the recorded physical strength and cognitive function information (Step S304).

Next, the server 1 generates the training instruction information, notifies the user of it, and records the result of the training implemented by the user (Step S306). Next, the server 1 predicts the future physical strength and cognitive function information in a case where the user is assumed to continue the implementation of the training on the basis of the history of the result of the implemented training and the change in the physical strength and cognitive function information caused by the implementation of the training (Step S308). Then, the server 1 calculates the insurance premium to be passed on in accordance with the improvement prediction of the physical strength and cognitive function (Step S310). For example, the server 1 calculates, as the insurance premium to be returned to the user, the difference between the insurance premium calculated in Step S304 and the insurance premium corresponding to the future physical strength and cognitive function information.

### <3.4. Supplement>

In the above description, the proposed technology is explained with car insurance as an example, but the application of the proposed technology is not limited to car insurance. For example, the proposed technology is applicable to any type of insurance such as life insurance or nursing insurance. However, the type of physical strength and cognitive function information used in calculation of the insurance risk is set for each type of insurance for which the insurance risk is calculated. The reaction rate, the lower limb cooperativeness, and the attentiveness are considered to be important in order to drive a car safely, for example. Therefore, when calculating the insurance risk related to the car insurance, the server 1 calculates the insurance risk and the insurance premium on the basis of the physical strength and cognitive function information related to the reaction rate, the lower limb cooperativeness, and the attentiveness. The insurance risk can be calculated more accurately by thus switching the physical strength and cognitive function information used for the calculation of the insurance risk for each type of insurance.

### <<4. Hardware Configuration Example>>

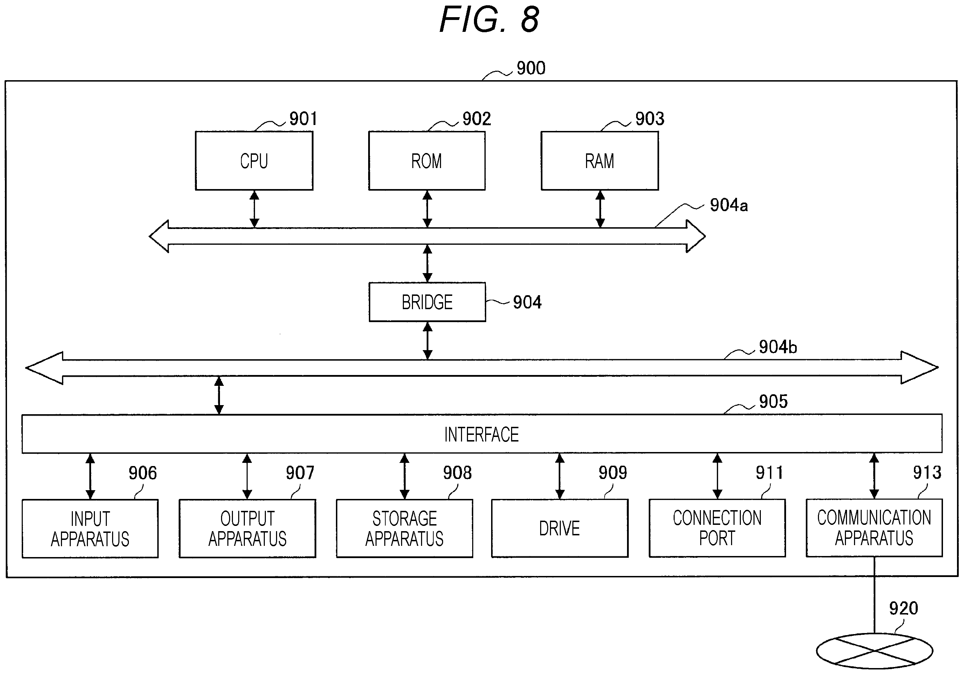

Lastly, the hardware configuration of the information processing apparatus according to the present embodiment will be described with reference to Fig. 8. Fig. 8 is a block diagram presenting an example of the hardware configuration of the information processing apparatus according to the present embodiment. It is to be noted that an information processing apparatus 900 presented in Fig. 8 can implement, for example, the server 1 presented in Fig. 3. Information processing by the server 1 according to the present embodiment is implemented by cooperation between software and hardware that is described below.

As presented in Fig. 8, the information processing apparatus 900 includes a central processing unit (CPU) 901, a read only memory (ROM) 902, a random access memory (RAM) 903, and a host bus 904a. In addition, the information processing apparatus 900 includes a bridge 904, an external bus 904b, an interface 905, an input apparatus 906, an output apparatus 907, a storage apparatus 908, a drive 909, a connection port 911, and a communication apparatus 913. The information processing apparatus 900 may have a processing circuit such as an electric circuit, a DSP, or an ASIC in place of or in addition to the CPU 901.

The CPU 901 functions as an arithmetic processing apparatus and a control apparatus, and controls the overall operation in the information processing apparatus 900 in accordance with various programs. Furthermore, the CPU 901 may also be a microprocessor. The ROM 902 stores programs, arithmetic parameters, and the like used by the CPU 901. The RAM 903 temporarily stores a program to be used in the execution of the CPU 901, parameters appropriately changing in the execution, and the like. The CPU 901 can form the control unit 130 presented in Fig. 3, for example.

The CPU 901, the ROM 902, and the RAM 903 are mutually connected by the host bus 904a including a CPU bus or the like. The host bus 904a is connected to the external bus 904b such as a peripheral component interconnect/interface (PCI) bus via the bridge 904. It is to be noted that the host bus 904a, the bridge 904, and the external bus 904b do not necessarily need to be configured separately, and these functions may be implemented on a single bus.

The input apparatus 906 is implemented by an apparatus to which information is input by the user, such as a mouse, a keyboard, a touch screen, a button, a microphone, a switch, or a lever, for example. In addition, the input apparatus 906 may be a remote control apparatus using infrared rays or other radio waves, for example, or may be external connection equipment such as a mobile phone or a PDA compatible with the operation of the information processing apparatus 900. Furthermore, the input apparatus 906 may include an input control circuit and the like that generates an input signal on the basis of information input by the user using the input means described above, for example, and outputs the input signal to the CPU 901. By operating the input apparatus 906, the user of the information processing apparatus 900 can input various types of data and instruct the processing operation to the information processing apparatus 900.

The output apparatus 907 is provided as an apparatus capable of visually or aurally notifying the user of the acquired information. Such apparatuses include display apparatuses such as a CRT display apparatus, a liquid crystal display apparatus, a plasma display apparatus, an EL display apparatus, a laser projector, an LED projector, and a lamp, audio output apparatuses such as a speaker and a headphone, a printer apparatus, and the like. The output apparatus 907 outputs results obtained by various processing performed by the information processing apparatus 900, for example. Specifically, the display apparatus visually displays, in various formats such as text, an image, a table, and a graph, results obtained by various processing performed by the information processing apparatus 900. On the other hand, the audio output apparatus converts an audio signal including reproduced audio data, acoustic data, and the like into an analog signal and aurally outputs the analog signal.

The storage apparatus 908 is an apparatus for data storage provided as an example of a storage unit of the information processing apparatus 900. The storage apparatus 908 is implemented by, for example, a magnetic storage device such as an HDD, a semiconductor storage device, an optical storage device, a magneto-optical storage device, or the like. The storage apparatus 908 may include a storage medium, a recording apparatus that records data in the storage medium, a reading apparatus that reads data from the storage medium, a deletion apparatus that deletes the data recorded in the storage medium, and the like. The storage apparatus 908 stores programs executed by the CPU 901, various types of data, various types of data acquired from the outside, and the like. The storage apparatus 908 can form the storage unit 120 presented in Fig. 3, for example.

The drive 909 is a reader/writer for a storage medium and is built in or attached to the information processing apparatus 900. The drive 909 reads information recorded in a mounted magnetic disk, an optical disk, a magneto-optical disk, or a removable storage medium such as a semiconductor memory, and outputs the information to the RAM 903. In addition, the drive 909 can also write information into the removable storage medium.

The connection port 911 is an interface connected to external equipment, and is a connection port with the external equipment capable of transmitting data by a universal serial bus (USB), for example.

The communication apparatus 913 is a communication interface provided by a communication device for connecting to a network 920, for example. The communication apparatus 913 is, for example, a communication card or the like for wired or wireless local area network (LAN), Long Term Evolution (LTE), Bluetooth (registered trademark), or Wireless USB (WUSB). In addition, the communication apparatus 913 may be a router for optical communication, a router for asymmetric digital subscriber line (ADSL), a modem for various communications, or the like. The communication apparatus 913 can transmit and receive signals and the like to and from the Internet or other communication equipment in accordance with a predetermined protocol such as TCP/IP, for example. The communication apparatus 913 can form the communication unit 110 presented in Fig. 3, for example.

It is to be noted that the network 920 is a wired or wireless transmission path for information transmitted from an apparatus connected to the network 920. For example, the network 920 may include a public line network such as the Internet, a telephone line network, a satellite communication network, various local area networks (LAN) including Ethernet (registered trademark), wide area networks (WAN), and the like. In addition, the network 920 may also include a leased line network such as an Internet protocol - virtual private network (IP-VPN) .

An example of the hardware configuration capable of implementing the function of the information processing apparatus 900 according to the present embodiment has been described above. Each of the components described above may be implemented by using a general-purpose member or may be implemented by hardware specialized in the function of each of the components. Accordingly, the hardware configuration to be used can be appropriately changed in accordance with the technical level at the time of carrying out the present embodiment.

It is to be noted that computer programs for implementing each of the functions of the information processing apparatus 900 according to the present embodiment as described above can be produced and mounted on a PC or the like. Furthermore, it is possible to also provide a computer-readable recording medium in which such computer programs are stored. The recording medium is, for example, a magnetic disk, an optical disk, a magneto-optical disk, a flash memory, or the like. In addition, the computer program described above may be distributed via a network, for example, without using a recording medium.

### <<5. Summary>>

An embodiment of the present disclosure has been described in detail above with reference to Figs. 1 to 8. As described above, the server 1 according to the present embodiment sets a correlation between the insurance risk and the physical strength and cognitive function information. This makes it possible to calculate the insurance risk and the insurance premium on the basis of the physical strength and cognitive function information of the user. In other words, the basis for an insurance scheme taking the physical strength and cognitive function into account is provided.

In addition, the server 1 according to the present embodiment calculates the insurance risk of the user on the basis of the set correlation and the physical strength and cognitive function information. This makes it possible to reflect the physical strength and cognitive function in the insurance risk. In particular, an increase in risk of a car accident due to the decline in the physical strength and cognitive function of the elderly person can be appropriately reflected in the insurance risk.

While the preferred embodiment of the present disclosure has been described in detail with reference to the accompanying drawings, the technical scope of the present disclosure is not limited to such an example. It is obvious that a person ordinarily skilled in the art of the present disclosure is capable of conceiving of various modifications or variations within the scope of the technical idea set forth in the claims, and these are also understood to naturally fall within the technical scope of the present disclosure.

In addition, the processing described in the present description using the flowcharts and sequence diagrams may not necessarily be executed in the order presented in the drawings. Some processing steps may be executed in parallel. Furthermore, additional processing steps may be employed, and some processing steps may be omitted.

In addition, the effects described in the present description are only exemplary or illustrative and not restrictive. That is, the technology according to the present disclosure can have other effects, as will be apparent to those skilled in the art from the description herein, in addition to or in place of the above effects.

It is to be noted that the following configuration also falls within the technical scope of the present disclosure.
(1) An information processing apparatus including
   a setting unit configured to set a correlation between an insurance risk and information indicating a physical strength and cognitive function.
(2) The information processing apparatus according to (1), further including:
   an acquisition unit configured to acquire information indicating the physical strength and cognitive function of a user; and
   a calculation unit configured to calculate an insurance risk of the user on the basis of the information indicating the physical strength and cognitive function of the user and the correlation.
(3) The information processing apparatus according to (2), in which the calculation unit calculates the insurance risk of the user on the basis of a prediction result of the information indicating the physical strength and cognitive function of the user.
(4) The information processing apparatus according to (3), in which the calculation unit calculates the insurance risk of the user on the basis of a prediction result of the information indicating the physical strength and cognitive function of the user corresponding to an implementation assumption of training for improvement of the physical strength and cognitive function.
(5) The information processing apparatus according to (4), in which the calculation unit calculates the insurance risk of the user on the basis of a prediction result of the information indicating the physical strength and cognitive function of the user in each of cases where the user implements the training for improving the physical strength and cognitive function and where the user does not implement the training for improvement of the physical strength and cognitive function.
(6) The information processing apparatus according to any one of (2) to (5), in which the calculation unit calculates the insurance risk of the user on the basis of a history of the information indicating the physical strength and cognitive function of the user.
(7) The information processing apparatus according to any one of (2) to (6), in which the calculation unit calculates the insurance risk of the user on the basis of a history of a result of training implemented by the user for improvement of the physical strength and cognitive function.
(8) The information processing apparatus according to any one of (2) to (7), further including a generation unit configured to generate information for instructing the user to implement the training for improvement of the physical strength and cognitive function, on the basis of the information indicating the physical strength and cognitive function of the user.
(9) The information processing apparatus according to any one of (1) to (8), in which a type of the information indicating the physical strength and cognitive function used in calculation of an insurance risk is set for each type of insurance for which an insurance risk is calculated.
(10) An information processing method including:
   setting, by a processor, a correlation between an insurance risk and information indicating a physical strength and cognitive function.

### REFERENCE SIGNS LIST

- 1: Server
- 2: User terminal
- 3: Measurement facility
- 4: Measurement apparatus
- 9: Network
- 100: Apparatus
- 110: Communication unit
- 120: Storage unit
- 130: Control unit
- 131: Setting unit
- 132: Acquisition unit
- 133: Prediction unit
- 134: Calculation unit
- 135: Training support unit

## Claims

1. An information processing apparatus comprising:
a setting unit configured to set a correlation between an insurance risk and information indicating a physical strength and cognitive function.

2. The information processing apparatus according to claim 1, further comprising:
an acquisition unit configured to acquire information indicating the physical strength and cognitive function of a user; and
a calculation unit configured to calculate an insurance risk of the user on a basis of the information indicating the physical strength and cognitive function of the user and the correlation.

3. The information processing apparatus according to claim 2, wherein the calculation unit calculates the insurance risk of the user on a basis of a prediction result of the information indicating the physical strength and cognitive function of the user.

4. The information processing apparatus according to claim 3, wherein the calculation unit calculates the insurance risk of the user on a basis of a prediction result of the information indicating the physical strength and cognitive function of the user corresponding to an implementation assumption of training for improvement of the physical strength and cognitive function.

5. The information processing apparatus according to claim 4, wherein the calculation unit calculates the insurance risk of the user on a basis of a prediction result of the information indicating the physical strength and cognitive function of the user in each of cases where the user implements the training for improving the physical strength and cognitive function and where the user does not implement the training for improvement of the physical strength and cognitive function.

6. The information processing apparatus according to claim 2, wherein the calculation unit calculates the insurance risk of the user on a basis of a history of the information indicating the physical strength and cognitive function of the user.

7. The information processing apparatus according to claim 2, wherein the calculation unit calculates the insurance risk of the user on a basis of a history of a result of training implemented by the user for improvement of the physical strength and cognitive function.

8. The information processing apparatus according to claim 2, further comprising a generation unit configured to generate information for instructing the user to implement the training for improvement of the physical strength and cognitive function, on a basis of the information indicating the physical strength and cognitive function of the user.

9. The information processing apparatus according to claim 1, wherein a type of the information indicating the physical strength and cognitive function used in calculation of an insurance risk is set for each type of insurance for which an insurance risk is calculated.

10. An information processing method comprising:
setting, by a processor, a correlation between an insurance risk and information indicating a physical strength and cognitive function.
